(19) Europäisches Patentamt / European Patent Office / Office européen des brevets

(11) **EP 3 340 184 A1**

(12) **EUROPEAN PATENT APPLICATION**

(43) Date of publication:
**27.06.2018 Bulletin 2018/26**

(51) Int Cl.:
**G06T 15/06** (2011.01)  **G06T 17/10** (2006.01)
**G06T 15/08** (2011.01)

(21) Application number: **17210078.6**

(22) Date of filing: **22.12.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **25.12.2016 US 201615390509**

(71) Applicant: **Biosense Webster (Israel) Ltd.
Yokneam 2066717 (IL)**

(72) Inventors:
• ZAR, Lior
  2066717 Yokneam (IL)
• KATZ, Natan Sharon
  2066717 Yokneam (IL)
• COHEN, Benjamin
  2066717 Yokneam (IL)

(74) Representative: **Carpmaels & Ransford LLP
One Southampton Row
London WC1B 5HA (GB)**

(54) **FAST RENDERING OF QUADRICS**

(57) Described embodiments include an apparatus that includes a display, including a screen, and a processor. The processor is configured to define a bounding region on the screen. The processor is further configured to render a quadric, which is defined in a parameter space, over a three-dimensional electroanatomical map of a surface of a heart that is displayed on the screen, by, for each pixel in the bounding region, transforming, to the parameter space, a virtual ray that passes through the pixel, ascertaining whether a point of intersection between the transformed virtual ray and the quadric exists in the parameter space, and, subsequently, provided the point of intersection exists, rendering the pixel on the screen, based on properties of the point of intersection. Other embodiments are also described.

46

DEFINE QUADRIC IN PARAMETER SPACE — 48

DEFINE BOUNDING REGION IN SCREEN SPACE — 50

SELECT NEXT PIXEL IN BOUNDING REGION — 52

TRANSFORM VIRTUAL RAY, WHICH PASSES THROUGH SELECTED PIXEL, TO PARAMETER SPACE — 54

ATTEMPT TO COMPUTE POINT OF INTERSECTION BETWEEN TRANSFORMED VIRTUAL RAY AND QUADRIC — 56

DOES POINT OF INTERSECTION EXIST? — 58
NO / YES

COMPUTE NORMAL AT POINT OF INTERSECTION — 60

TRANSFORM NORMAL TO VIEW SPACE — 62

RENDER SELECTED PIXEL BASED ON TRANSFORMED NORMAL AND COLORING AT POINT OF INTERSECTION — 64

MORE PIXELS IN BOUNDING REGION? — 66
YES / NO

END

FIG. 4

EP 3 340 184 A1

## Description

### FIELD OF THE INVENTION

The present invention relates to the field of computer graphics, and especially to the rendering of quadrics.

### BACKGROUND

In a rendering technique known as ray casting, a computer simulates the casting of rays onto a virtual scene, and renders the scene in accordance with the interaction between these rays and objects in the scene.

In some rendering applications, objects in the virtual scene are modeled as quadratic surfaces, or "quadrics." The equation for a quadric may be expressed in homogenous (x,y,z,w) coordinates as $Ax^2 + 2Bxy + 2Cxz + 2Dxw + Ey^2 + 2Fyz + 2Gyw + Hz^2 + 2Izw + Jw^2 = 0$. More compactly, this may be written in matrix form as $X^TQX = 0$, where $X = \begin{matrix} x \\ y \\ z \\ w \end{matrix}$ and

$$Q = \begin{matrix} A & B & C & D \\ B & E & F & G \\ C & F & H & I \\ D & G & I & J \end{matrix}.$$ (Generally, w is set to 1.)

Examples of quadric surfaces include spheres, ellipsoids, cylinders, cones, hyperbolic paraboloids, paraboloids, and hyperboloids.

A ray may be expressed by the equation $R = O + tD$, where R is any point on the ray, O is the origin of the ray, D is the direction vector of the ray, and t is a scalar parameter. R, O, and D may be expressed in homogenous coordinates.

Sigg, Christian, et al., "GPU-Based Ray-Casting of Quadratic Surfaces," SPBG, 2006, which is incorporated herein by reference, proposes an efficient rendering technique for quadric primitives based on GPU-accelerated splatting.

### SUMMARY OF THE INVENTION

There is provided, in accordance with some embodiments of the present invention, apparatus that includes a display, including a screen, and a processor. The processor is configured to define a bounding region on the screen. The processor is further configured to render a quadric, which is defined in a parameter space, over a three-dimensional electroanatomical map of a surface of a heart that is displayed on the screen, by, for each pixel in the bounding region, transforming, to the parameter space, a virtual ray that passes through the pixel, ascertaining whether a point of intersection between the transformed virtual ray and the quadric exists in the parameter space, and, subsequently, provided the point of intersection exists, rendering the pixel on the screen, based on properties of the point of intersection.

In some embodiments, the processor is further configured to define the quadric, in the parameter space, such that the quadric is bounded by a cube having eight corners, two of which are at (-1,-1,-1) and (1,1,1), respectively.

In some embodiments, the processor is configured to define the bounding region by:

transforming the corners of the cube to a screen space, which is defined in terms of a coordinate system of the screen, and
defining the bounding region such that the bounding region is a minimum bounding rectangle of the transformed corners.

In some embodiments, the processor is further configured to define the quadric such that the quadric is representable by a 4x4 diagonal matrix Q.

In some embodiments,
the virtual ray has a ray origin O and a ray-direction vector D,
the processor is configured to transform the virtual ray by computing O', which is the ray origin O transformed to the parameter space, and D', which is the ray-direction vector D transformed to the parameter space, and
the processor is configured to ascertain whether the point of intersection exists by attempting to compute the point of intersection, by:

computing a first coefficient $a = D'^TQD'$, where $D'^T$ is a transpose of D', a second coefficient $b = 2D'^TQO'$, and a third coefficient $c = O'QO'$, and subsequently, solving, for a parameter t, $at^2 + bt + c = 0$.

In some embodiments, the processor is further configured to represent Q as a four-element vector $Q_D$, and the processor is configured to compute each of the first coefficient a, the second coefficient b, and the third coefficient c by performing an element-wise multiplication of $Q_D$.

In some embodiments, the processor is further configured to receive a signal that indicates a location of a distal end of an intrabody catheter, and the processor is configured to render the quadric over a portion of the three-dimensional electroanatomical map that corresponds to the indicated location.

In some embodiments, the processor is configured to render the quadric in response to an ablating signal being passed into the surface of the heart, by the distal end of the intrabody catheter, at the indicated location.

In some embodiments, the processor is configured to render the pixel on the screen by:

computing a normal vector to the quadric at the point of intersection, and

rendering the pixel, based on a coloring of the quadric at the point of intersection, and the normal vector.

There is further provided, in accordance with some embodiments of the present invention, a method that includes, using a processor, defining a bounding region on a screen. The method further includes rendering a quadric, which is defined in a parameter space, over a three-dimensional electroanatomical map of a surface of a heart that is displayed on the screen, by, for each pixel in the bounding region, transforming, to the parameter space, a virtual ray that passes through the pixel, ascertaining whether a point of intersection between the transformed virtual ray and the quadric exists in the parameter space, and, subsequently, provided the point of intersection exists, rendering the pixel on the screen, based on properties of the point of intersection.

There is further provided, in accordance with some embodiments of the present invention, a computer software product including a tangible non-transitory computer-readable medium in which program instructions are stored. The instructions, when read by a processor, cause the processor to define a bounding region on a screen. The instructions, when read by the processor, further cause the processor to render a quadric, which is defined in a parameter space, over a three-dimensional electroanatomical map of a surface of a heart that is displayed on the screen, by, for each pixel in the bounding region, transforming, to the parameter space, a virtual ray that passes through the pixel, ascertaining whether a point of intersection between the transformed virtual ray and the quadric exists in the parameter space, and, subsequently, provided the point of intersection exists, rendering the pixel on the screen, based on properties of the point of intersection.

The present invention will be more fully understood from the following detailed description of embodiments thereof, taken together with the drawings, in which:

## BRIEF DESCRIPTION OF THE DRAWINGS

Fig. 1 is a schematic illustration of a system for rendering quadrics over an electroanatomical map, in accordance with some embodiments of the present invention;

Fig. 2 is a schematic overview of a method for rendering a quadric, in accordance with some embodiments of the present invention;

Fig. 3 is a schematic illustration of aspects of a method for rendering a quadric, in accordance with some embodiments of the present invention; and

Fig. 4 is a flow diagram for a rendering method performed by a processor, in accordance with some embodiments of the present invention.

## DETAILED DESCRIPTION OF EMBODIMENTS

OVERVIEW

In embodiments of the present invention, an electroanatomical map of a portion of a subject's heart is constructed, and is then rendered on-screen. (Such a map is typically embodied by a three-dimensional mesh that is constructed from a plurality of points that correspond to respective locations at which an electrical property was measured.) A plurality of quadrics may then be rendered over the electroanatomical map. For example, during and/or following an ablation procedure, each portion of cardiac tissue that has been ablated, or is presently being ablated, may be marked on-screen by a respective quadric, such as a sphere or an ellipse. Typically, the resolution of this marking is relatively high, such that, in some cases, it may be necessary to render a large number (e.g., tens of thousands) of quadrics on-screen.

Embodiments of the present invention provide improved methods and systems for rendering quadrics, such that a large number of quadrics may be quickly and accurately rendered. For example, the following algorithm may be used to render each of the quadrics:

(i) Define a diagonal matrix Q, which represents a quadric in parameter space (or "model space") that is centered at the origin and is bounded by a cube with corners at (-1,-1,-1) and (1,1,1). For example, a unit sphere of radius 1, centered at (0,0,0), is represented by

$$Q = \begin{pmatrix} 1 & 0 & 0 & 0 \\ 0 & 1 & 0 & 0 \\ 0 & 0 & 1 & 0 \\ 0 & 0 & 0 & -1 \end{pmatrix}.$$

(ii) Compute the matrix M, which, upon multiplying a point having homogenous coordinates $\begin{pmatrix} x \\ y \\ z \\ 1 \end{pmatrix}$ in parameter space, transforms the point such that the point is properly placed within the virtual scene, which exists in "world space." The matrix M thus represents a transformation from parameter space to world space.

(iii) Given the matrix V, which represents a transformation from world space to view space, and P, which represents a perspective or orthographic projection from view space to screen space (which is defined in terms of the coordinate system of the screen on which the quadric is rendered), compute the matrix T = PVM, which represents a transformation from parameter space to screen space. Also, compute $T^{-1}$, the inverse of T, which represents a transformation from screen space to parameter space.

(iv) Define, on the screen (i.e., in screen space), a bounding region B that contains all of the pixels in which the quadric might be rendered. Typically, B is a bounding rectangle. (Some graphics processing units may require that this bounding rectangle be divided into two triangles.) Since the quadric is bounded in parameter space by the above-described cube, B may be computed by transforming the eight corners of the cube to screen space, and then finding the minimum bounding rectangle of these transformed corners.

(v) Perform the following steps for each pixel in B:

(v-a) From the matrix P, compute D and O, where O is the origin of a ray that passes through the pixel, and D is the direction vector of this ray. (For an orthographic projection, O, will vary between the pixels, with D constant. For a perspective projection, D will vary between the pixels, with O constant.) Then transform O and D to parameter space, by multiplying each of these vectors by $T^{-1}$. The transformed ray may be expressed as R' = O' + tD', where O' = $T^{-1}$O, and D' = $T^{-1}$D.

(v-b) In parameter space, find any points X' at which the transformed ray intersects the quadric. (If the ray does not intersect the quadric, move on to the next pixel in B.) If there is more than one point of intersection, choose the point X' that the ray collides with first. Also calculate the normal vector N' to the quadric at X'.

(v-c) Transform N' to view space, by multiplying N' by a "normal matrix" $M_N$ that is derived from V (N = $M_N$N').

(v-d) Calculate the coloring of the pixel, based on the coloring of the quadric at point X' and the transformed normal vector N.

(It is noted that a plurality of pixels in bounding rectangle B may be processed, as described above, in parallel.)

An advantage of this algorithm is that, as described above, step (v-b) operates in parameter space, where the quadric is represented by a diagonal matrix Q. Since Q is diagonal, Q may be reduced to vector form. For example, assuming the notation for Q assumed above, a diagonal matrix Q may be represented by the vector $Q_d = \begin{bmatrix} A \\ E \\ H \\ J \end{bmatrix}$, $Q_d$ being the diagonal of Q. The representation of Q in this manner leads to a faster computation of the points of intersection X'. In contrast, were step (v-b) to operate in view space, the computation would be slower, given that the multiplication of Q by M, V, and/or any other transformational matrix typically causes Q to cease to be a diagonal matrix.

More particularly, substituting the transformed ray equation R' = O' + tD' into the quadric equation $X^TQX$ = 0, one arrives at the quadratic equation $at^2 + bt + c = 0$, where a = $D'^TQD'$, b = $2D'^TQO'$, and c = $O'^TQO'$. Hence, to find the intersections of the ray with the quadric, a, b, and c must be computed, following which the intersections may be found by solving for the roots of the above quadratic equation. A challenge, however, is that the computation of a, b, and c may involve a relatively large number of operations. For example, the computation of QO' (for the computation of b and c) includes computing the inner product of each of the rows of Q with O', which involves a total of 16 multiplication operations and 12 addition operations.

Embodiments of the present invention address this challenge, by solving for the intersections in parameter space, where Q is known to be a diagonal matrix. Given the diagonality of Q, $Q_d$ may be used to compute a, b, and c, which leads to a faster computation. For example, the computation of QO' may be performed by performing an element-wise multiplication of $Q_d$ with O', which involves only four multiplication operations.

Moreover, since Q is bounded by the cube with corners at (-1,-1,-1) and (1,1,1), various operations may be performed more quickly. For example, as described above, bounding region B may be computed relatively quickly, based on the projection of the corners of the cube onto the screen.

Embodiments described herein may be applied, for example, to the rendering of a sphere, ellipsoid, cylinder, cone, or hyperboloid, each of which may be represented by a diagonal matrix Q. Embodiments described herein may also be applied to the rendering of a paraboloid, in that, even though a paraboloid is not directly representable by a diagonal matrix, a paraboloid may be obtained by clipping an ellipsoid. Clipping may also be used to confine certain quadrics - such as cylinders, which extend to infinity - to the above-described cube in parameter space.

Although the present application relates to the rendering of quadrics mainly in the context of electroanatomical maps, it is noted that embodiments described herein may be applied to any suitable quadric-rendering application.

SYSTEM DESCRIPTION

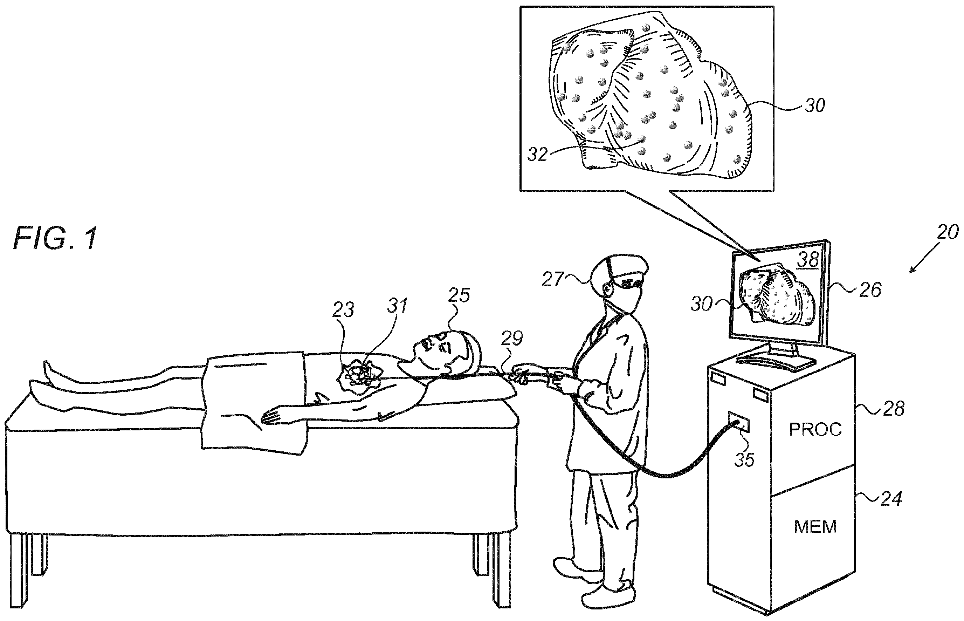

Reference is initially made to Fig. 1, which is a schematic illustration of a system 20 for rendering quadrics over an electroanatomical map, in accordance with some embodiments of the present invention. One commercial product embodying elements of system 20 is the CARTO® 3 System, available from Biosense Webster, Inc. This system may be modified by those skilled in the art to embody the principles of embodiments described herein.

Fig. 1 illustrates an ablation procedure performed on a heart 23 of a subject 25, using an intrabody catheter 29. Catheter 29 comprises a distal end 31, comprising one or more ablating electrodes, and one or more tracking sensors (e.g., electromagnetic tracking sensors), which track the location of distal end 31. During the procedure, as a physician 27 moves distal end 31 along a surface (e.g., the inner or epicardial surface) of heart 23, the ablating electrodes pass ablating signals into the surface. While the procedure is ongoing, a processor (PROC) 28 receives, via an electrical interface 35 (comprising, for example, a port or other connector), signals from the tracking sensors, which indicate the locations of distal end 31. Processor 28 stores these locations in a computer memory (MEM) 24.

During, and/or following, the ablation procedure, processor 28 retrieves, from computer memory 24, a three-dimensional electroanatomical map 30 of the cardiac surface, and then renders map 30 on a screen 38 of a display 26. As described in detail below, the processor further renders a plurality of quadrics 32 over the map. Typically, each of the quadrics is rendered over a respective portion of the map that correspond to a location of distal end 31 during the procedure, as indicated by the location sensors. For example, a respective quadric may mark each location at which the distal end of the catheter ablated tissue of the subject. In other words, the processor may render quadrics over respective portions of the map corresponding to respective locations of distal end 31, in response to ablating signals being passed into the surface of the heart, by distal end 31, at the respective locations.

The rendering of quadrics over map 30, as described herein, may be performed in real-time, to help guide the physician during the procedure, and/or following the procedure, to facilitate a post-procedural assessment.

In general, processor 28 may be embodied as a single processor, or as a cooperatively networked or clustered set of processors. Processor 28 is typically a programmed digital computing device comprising a central processing unit (CPU), random access memory (RAM), non-volatile secondary storage, such as a hard drive or CD ROM drive, network interfaces, and/or peripheral devices. Program code, including software programs, and/or data are loaded into the RAM for execution and processing by the CPU and results are generated for display, output, transmittal, or storage, as is known in the art. The program code and/or data may be downloaded to the processor in electronic form, over a network, for example, or it may, alternatively or additionally, be provided and/or stored on non-transitory tangible media, such as magnetic, optical, or electronic memory. Such program code and/or data, when provided to the processor, produce a machine or special-purpose computer, configured to perform the tasks described herein.

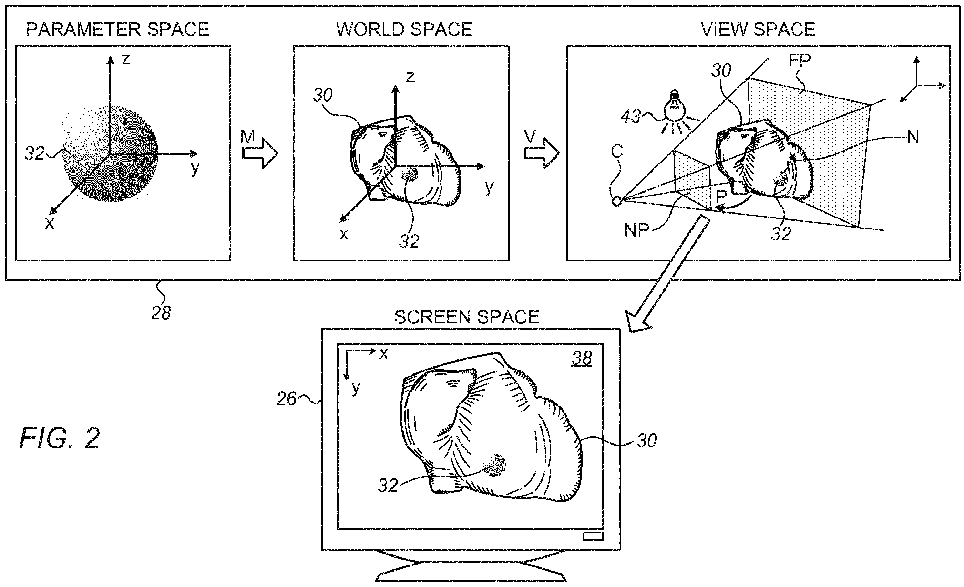

Reference is now made to Fig. 2, which is a schematic overview of a method for rendering a quadric 32, in accordance with some embodiments of the present invention.

Fig. 2 depicts several "spaces," each of which is defined in terms of a respective coordinate system. Several of these spaces are virtual spaces that are defined by processor 28. In particular:

(i) Parameter space is a virtual three-dimensional space in which virtual objects to be rendered are defined in isolation.

(ii) World space is a virtual three-dimensional space in which objects defined in parameter space are suitably oriented, sized, and placed with respect to other objects in the virtual scene that is to be rendered. A matrix M transforms objects from parameter space to world space, by rotating, scaling, and/or translating these objects.

(iii) View space is a virtual three-dimensional space in which the virtual scene from world space, while being illuminated by a virtual light source 43, is imaged by a virtual camera, located at a camera location C. First, the virtual scene is placed between the near plane NP of the camera and the far plane FP of the camera (which may be located at infinite distance from the camera), in accordance with a transformation matrix V, which transforms the virtual scene from world space. Then, the processor causes the virtual camera to image the virtual scene, by projecting the scene onto near plane NP in accordance with a projection matrix P.

Near plane NP of the virtual camera is represented, in the real world, by screen 38; in other words, the virtual scene is rendered on screen 38 in accordance with the view of the virtual camera in view space. Coordinates of pixels belonging to screen 38 are defined in terms of a two-dimensional screen space. The above-described projection matrix P determines the location in screen space at which an object in view space is rendered, as well as the size and orientation of the rendered object. (Hence, projection matrix P may be said to represent a transformation from view space to screen space, as described above in the Overview.) The color with which the object is rendered is determined by the "objective" color of the object (as defined, typically, in parameter space), relevant properties of (such as the position and intensity of) virtual light source 43, and the normal vector N to the object, which affects the manner in which the object is illuminated by the virtual light source.

In accordance with the particular ray-casting techniques described below, the projection of objects onto near plane NP (and hence onto the screen) is accomplished by passing a plurality of virtual rays through the near plane (or equivalently, through the screen). Each of these virtual rays has a ray origin, which coincides with camera location C, and a ray-direction vector, each of which is derived from projection matrix P. As the virtual rays collide with objects in the virtual scene, the objects are projected onto the near plane, and are hence rendered on-screen.

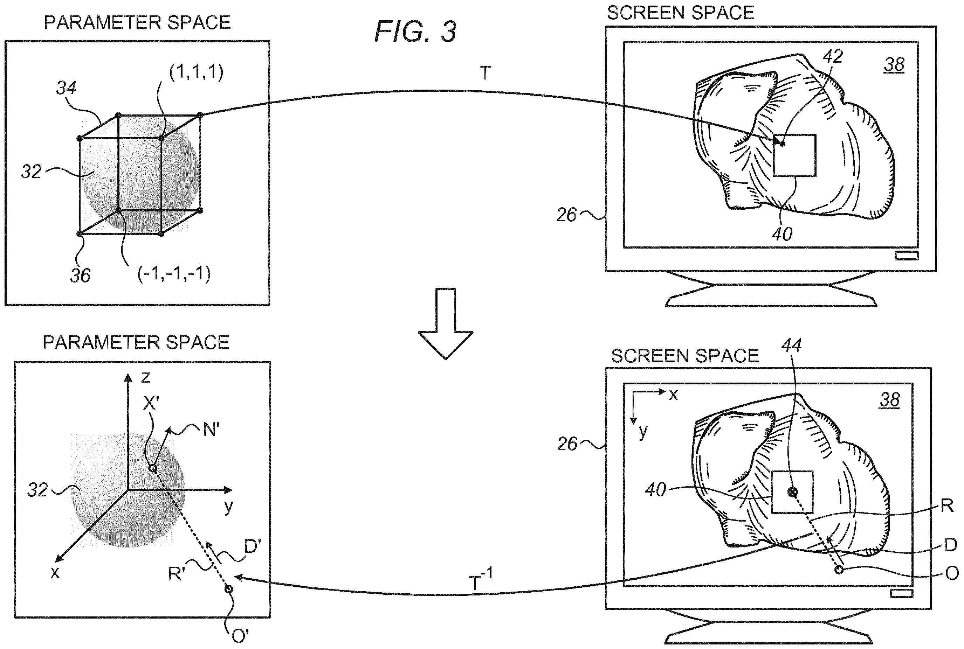

Fig. 2 shows a particular example, whereby the processor defines a quadric 32 in parameter space, transforms the quadric to world space such that the quadric is placed over an electroanatomical map 30, images map 30 and quadric 32 in view space, and, finally, renders map 30 and quadric 32 on-screen. Aspects of this process are described in more detail immediately below, with reference to Fig. 3.

Reference is now made to Fig. 3, which is a schematic illustration of aspects of a method for rendering a quadric, in accordance with some embodiments of the present invention.

As described above with reference to Fig. 2, the processor first defines quadric 32 in parameter space, which is defined in terms of an (x,y,z) coordinate system. As described above in the Overview, the quadric is typically defined such that it is bounded by a cube 34 having eight corners 36, two of which are at (-1,-1,-1) and (1,1,1), respectively. As further described above in the Overview, the quadric is typically defined such that it is representable by a 4x4 diagonal matrix Q. For example, as shown in Fig. 3, the processor may define a sphere of radius 1 centered at the origin, which is representable by the di-

agonal matrix $Q = \begin{pmatrix} 1 & 0 & 0 & 0 \\ 0 & 1 & 0 & 0 \\ 0 & 0 & 1 & 0 \\ 0 & 0 & 0 & -1 \end{pmatrix}$. In addition to de-

fining the shape and size of the quadric, the processor defines the coloring of the quadric. For example, the processor may make the quadric uniformly colored. (Typically, the quadric is defined in accordance with values stored in memory 24, and/or in accordance with relevant input from a user.)

As further shown in Fig. 3, display 26 comprises a screen 38, comprising a plurality of pixels, each of which has (x,y) coordinates within the screen space of the screen. To render quadric 32 on the screen 38, the processor first defines a bounding region 40 on the screen, i.e., in screen space. (Typically, the defined bounding region is not displayed on the screen.) As described above in the Overview, to define bounding region 40, the processor typically first transforms corners 36 of the cube to screen space. This is shown, in Fig. 3, for one of the corners 36, whereby the matrix T, upon multiplying the

parameter-space $\begin{pmatrix} x \\ y \\ z \\ 1 \end{pmatrix}$ coordinates of the corner, transforms the corner to its new (x,y) coordinates in screen space, indicated in Fig. 3 by a transformed corner 42. After thus transforming each of corners 36 to screen space, the processor defines bounding region 40 such that the bounding region is a minimum bounding rectangle of the transformed corners 42.

Next, as shown in the lower portion of Fig. 3, the processor iterates over each pixel 44 in bounding region 40, and, if appropriate, renders the pixel as part of the quadric. In particular, for each pixel 44, the processor first transforms, from the screen space to the parameter space, a virtual ray R that passes through the pixel. As described above in the Overview and with reference to Fig. 2, virtual ray R has a ray origin O, which is located in front of the screen (at the position of the virtual camera in view space), and a ray-direction vector D, which describes the direction, into the screen, of the virtual ray. (As described above, the virtual ray is thus described by the equation R = O + tD.) The processor transforms this virtual ray by computing O', which is the ray origin O transformed to parameter space, and D', which is the ray-direction vector D transformed to parameter space. To compute O' and D', the processor multiplies O and D, respectively, by $T^{-1}$, the inverse of the transformation matrix T.

The transformation of the virtual ray yields a transformed virtual ray R' in parameter space, described by the equation R' = O' + tD'. Following this transformation, the processor ascertains whether a point of intersection between the transformed virtual ray and the quadric exists in the parameter space, by attempting to compute a point of intersection X' between transformed virtual ray R' and quadric 32. In other words, the processor attempts to identify a point X' in parameter space at which the transformed virtual ray collides with the quadric. Provided that such a point of intersection X' exists, the processor then renders pixel 44 on screen 38, as further described below, based on properties of point of intersection X', such as the color of the quadric, and normal to the quadric, at point X'.

As described above in the Overview, the computation of point of intersection X' is relatively quick, given that the processor performs this computation in parameter space. For example, the processor may quickly compute each of the coefficients $a = D'^T Q D'$, $b = 2D'^T Q O'$, and $c = O'^T Q O'$, by representing Q as a four-element vector $Q_D$, and then performing an element-wise multiplication of $Q_D$. The processor may then solve, for the parameter t, the equation $at^2 + bt + c = 0$. Writing this solution (or "root") as $t_R$, the point of intersection X may then be computed as $O' + t_R D'$. (Typically, the equation $at^2 + bt + c = 0$ will have two positive roots, indicating that the virtual ray intersects the quadric at two points. In such a case, the processor chooses the point of intersection that is closer to the origin of the ray.)

For example, the processor may represent the unit sphere by $Q_D = \begin{matrix} 1 \\ & 1 \\ & & 1 \\ & & & -1 \end{matrix}$, which is the diagonal of the matrix Q for the unit sphere. Then, assuming that

$$O' = \begin{matrix} Ox \\ Oy \\ Oz \\ Ow \end{matrix} \text{ and } D' = \begin{matrix} Dx \\ Dy \\ Dz \\ Dw \end{matrix},$$ the processor may compute the first coefficient a by performing an element-wise multiplication of $Q_D$ with D', yielding the vector $\begin{matrix} Dx \\ Dy \\ Dz \\ -Dw \end{matrix}$, and then left-multiplying this vector by $D'^T$, yielding $Dx^2 + Dy^2 + Dz^2 - Dw^2$. Likewise, for the second coefficient b and third coefficient c, the processor may perform an element-wise multiplication of $Q_D$ with O', yielding the vector $\begin{matrix} Ox \\ Oy \\ Oz \\ -Ow \end{matrix}$, and then left-multiply this vector by $2D'^T$ and $O'^T$, respectively. The processor may then solve the equation $at^2 + bt + c = 0$, as described above.

Typically, along with computing the point of intersection X', the processor computes the normal vector N' to the quadric at the point of intersection. Then, following the computation of the point of intersection X' and the corresponding normal vector N', the processor transforms normal vector N' to view space, by left-multiplying N' by VM, yielding a transformed normal vector N. The processor then renders pixel 44, based on the coloring of the quadric at the point of intersection X', and the transformed normal vector N (which, as described above with reference to Fig. 2, influences the manner in which light from the virtual light source interacts with the quadric).

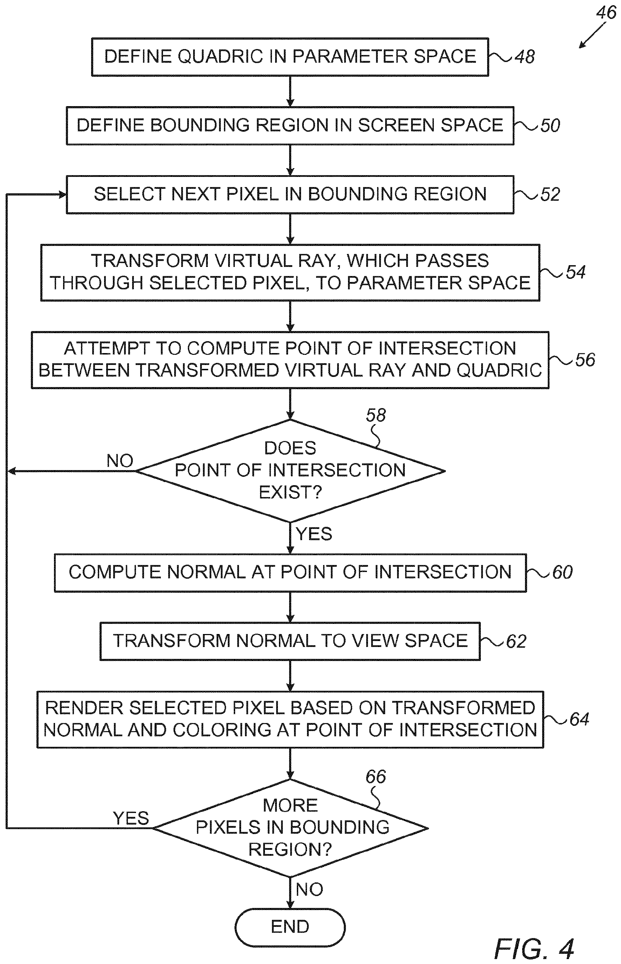

Reference is now made to Fig. 4, which is a flow diagram for a rendering method 46 performed by processor 28, in accordance with some embodiments of the present invention. (In general, the various steps of method 46 were already described above, but are again presented in brief with reference to Fig. 4.)

First, at a quadric-defining step 48, the processor defines the quadric, which is to be rendered, in parameter space. Next, at a bounding-region-defining step 50, the processor defines a suitable bounding region in screen space. This bounding region is typically the smallest region that can be computed within a reasonable amount of time and that contains all of the pixels that might correspond to a portion of the quadric. For example, as described above with reference to Fig. 3, the bounding region may be computed as the minimum bounding rectangle of the transformed corners of a cube that contains the quadric in parameter space.

Next, the processor begins to iterate over all of the pixels in the bounding region. At the start of each iteration, at a pixel-selecting step 52, the processor selects a pixel that has not yet been processed. Next, at a ray-transforming step 54, the processor transforms a virtual ray, which passes through the selected pixel, to parameter space. Subsequently, at an intersection-point-computing step 56, the processor attempts to compute a point of intersection between the transformed virtual ray and the quadric. For example, as described above in the Overview and with reference to Fig. 3, the processor may solve for the roots of the equation $at^2 + bt + c = 0$. Next, at a first checking step 58, the processor checks if the attempt was successful, i.e., if a point of intersection actually exists. If yes, the processor proceeds with the steps described below. Otherwise (e.g., if $at^2 + bt + c = 0$ has only imaginary roots), the processor does not render the selected pixel as part of the quadric, and instead selects the next pixel in the bounding region, at pixel-selecting step 52.

Following (or in conjunction with) the computation of the point of intersection, the processor, at a normal-computing step 60, computes the normal to the quadric at the point of intersection. Next, at a normal-transforming step 62, the processor transforms the normal to view space. Finally, at a rendering step 64, the processor renders the selected pixel, based on the transformed normal and the coloring of the quadric at the point of intersection.

Following the rendering of the pixel, the processor, at a second checking step 66, checks if more unprocessed pixels remain in the bounding region. If yes, the processor processes the next pixel. Otherwise, method 46 ends. (If additional quadrics are to be rendered, however, method 46 may be repeated for each of the additional quadrics.)

As described above in the Overview, in some embodiments, multiple pixels in the bounding region are processed in parallel. For example, all of the pixels in the bounding region may be processed in parallel, such that pixel-selecting step 52 is performed no more than once by each thread that is executed by the processor, and second checking step is not necessarily performed at all.

It will be appreciated by persons skilled in the art that the present invention is not limited to what has been particularly shown and described hereinabove. Rather, the scope of embodiments of the present invention includes both combinations and subcombinations of the various features described hereinabove, as well as variations and modifications thereof that are not in the prior art, which would occur to persons skilled in the art upon reading the foregoing description. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

**Claims**

1. Apparatus, comprising:

   a display, comprising a screen; and
   a processor, configured:

      to define a bounding region on the screen, and
      to render a quadric, which is defined in a parameter space, over a three-dimensional electroanatomical map of a surface of a heart that is displayed on the screen, by, for each pixel in the bounding region:

         transforming, to the parameter space, a virtual ray that passes through the pixel,
         ascertaining whether a point of intersection between the transformed virtual ray and the quadric exists in the parameter space, and
         subsequently, provided the point of intersection exists, rendering the pixel on the screen, based on properties of the point of intersection.

2. The apparatus according to claim 1, wherein the processor is further configured to define the quadric, in the parameter space, such that the quadric is bounded by a cube having eight corners, two of which are at (-1,-1,-1) and (1,1,1), respectively.

3. The apparatus according to claim 2, wherein the processor is configured to define the bounding region by:

   transforming the corners of the cube to a screen space, which is defined in terms of a coordinate system of the screen, and
   defining the bounding region such that the bounding region is a minimum bounding rectangle of the transformed corners.

4. The apparatus according to claim 1, wherein the processor is further configured to define the quadric such that the quadric is representable by a 4x4 diagonal matrix Q.

5. The apparatus according to claim 4,
   wherein the virtual ray has a ray origin O and a ray-direction vector D,
   wherein the processor is configured to transform the virtual ray by computing O', which is the ray origin O transformed to the parameter space, and D', which is the ray-direction vector D transformed to the parameter space, and
   wherein the processor is configured to ascertain whether the point of intersection exists by attempting to compute the point of intersection, by:

      computing a first coefficient $a = D'^T QD'$, where $D'^T$ is a transpose of D', a second coefficient $b = 2D'^T QO'$, and a third coefficient $c = O'QO'$, and subsequently, solving, for a parameter t, $at^2 + bt + c = 0$.

6. The apparatus according to claim 5, wherein the processor is further configured to represent Q as a four-element vector $Q_D$, and wherein the processor is configured to compute each of the first coefficient a, the second coefficient b, and the third coefficient c by performing an element-wise multiplication of $Q_D$.

7. The apparatus according to claim 1, wherein the processor is further configured to receive a signal that indicates a location of a distal end of an intrabody catheter, and wherein the processor is configured to render the quadric over a portion of the three-dimensional electroanatomical map that corresponds to the indicated location.

8. The apparatus according to claim 7, wherein the processor is configured to render the quadric in response to an ablating signal being passed into the surface of the heart, by the distal end of the intrabody catheter, at the indicated location.

9. The apparatus according to claim 1, wherein the processor is configured to render the pixel on the screen by:

   computing a normal vector to the quadric at the point of intersection, and
   rendering the pixel, based on a coloring of the quadric at the point of intersection, and the normal vector.

10. A method, comprising:

   using a processor, defining a bounding region on a screen; and
   rendering a quadric, which is defined in a parameter space, over a three-dimensional electroanatomical map of a surface of a heart that is displayed on the screen, by, for each pixel in the bounding region:

> transforming, to the parameter space, a virtual ray that passes through the pixel, ascertaining whether a point of intersection between the transformed virtual ray and the quadric exists in the parameter space, and subsequently, provided the point of intersection exists, rendering the pixel on the screen, based on properties of the point of intersection.

11. The method according to claim 10, further comprising defining the quadric, in the parameter space, such that the quadric is bounded by a cube having eight corners, two of which are at (-1,-1,-1) and (1,1,1), respectively.

12. The method according to claim 11, wherein defining the bounding region comprises:

> transforming the corners of the cube to a screen space, which is defined in terms of a coordinate system of the screen, and defining the bounding region such that the bounding region is a minimum bounding rectangle of the transformed corners.

13. The method according to claim 10, further comprising defining the quadric such that the quadric is representable by a 4x4 diagonal matrix Q.

14. The method according to claim 13, wherein the virtual ray has a ray origin O and a ray-direction vector D, wherein transforming the virtual ray comprises transforming the virtual ray by computing O', which is the ray origin O transformed to the parameter space, and D', which is the ray-direction vector D transformed to the parameter space, and wherein ascertaining whether the point of intersection exists comprises attempting to compute the point of intersection, by:

> computing a first coefficient $a = D'^{T}QD'$, where $D'^{T}$ is a transpose of D', a second coefficient $b = 2D'^{T}QO'$, and a third coefficient $c = O'QO'$, and subsequently, solving, for a parameter t, $at^2 + bt + c = 0$.

15. The method according to claim 14, further comprising representing Q as a four-element vector $Q_D$, wherein computing the first coefficient a, the second coefficient b, and the third coefficient c comprises computing each of the first coefficient a, the second coefficient b, and the third coefficient c by performing an element-wise multiplication of $Q_D$.

16. The method according to claim 10, further comprising receiving a signal that indicates a location of a distal end of an intrabody catheter, wherein rendering the quadric comprises rendering the quadric over a portion of the three-dimensional electroanatomical map that corresponds to the indicated location.

17. The method according to claim 16, wherein rendering the quadric comprises rendering the quadric in response to an ablating signal being passed into the surface of the heart, by the distal end of the intrabody catheter, at the indicated location.

18. The method according to claim 10, wherein rendering the pixel on the screen comprises:

> computing a normal vector to the quadric at the point of intersection, and rendering the pixel, based on a coloring of the quadric at the point of intersection, and the normal vector.

19. A computer software product comprising a tangible non-transitory computer-readable medium in which program instructions are stored, which instructions, when read by a processor, cause the processor:

> to define a bounding region on a screen, and to render a quadric, which is defined in a parameter space, over a three-dimensional electroanatomical map of a surface of a heart that is displayed on the screen, by, for each pixel in the bounding region:
>
>> transforming, to the parameter space, a virtual ray that passes through the pixel, ascertaining whether a point of intersection between the transformed virtual ray and the quadric exists in the parameter space, and subsequently, provided the point of intersection exists, rendering the pixel on the screen, based on properties of the point of intersection.

20. The computer software product according to claim 19, wherein the instructions further cause the processor:

> to compute a normal vector to the quadric at the point of intersection, and to render the pixel, based on a coloring of the quadric at the point of intersection, and the normal vector.

FIG. 1

PARAMETER SPACE

WORLD SPACE

VIEW SPACE

SCREEN SPACE

FIG. 2

EP 3 340 184 A1

FIG. 3

PARAMETER SPACE

SCREEN SPACE

PARAMETER SPACE

SCREEN SPACE

EP 3 340 184 A1

46

DEFINE QUADRIC IN PARAMETER SPACE — 48

DEFINE BOUNDING REGION IN SCREEN SPACE — 50

SELECT NEXT PIXEL IN BOUNDING REGION — 52

TRANSFORM VIRTUAL RAY, WHICH PASSES THROUGH SELECTED PIXEL, TO PARAMETER SPACE — 54

ATTEMPT TO COMPUTE POINT OF INTERSECTION BETWEEN TRANSFORMED VIRTUAL RAY AND QUADRIC — 56

58
DOES POINT OF INTERSECTION EXIST?

NO

YES

COMPUTE NORMAL AT POINT OF INTERSECTION — 60

TRANSFORM NORMAL TO VIEW SPACE — 62

RENDER SELECTED PIXEL BASED ON TRANSFORMED NORMAL AND COLORING AT POINT OF INTERSECTION — 64

66
MORE PIXELS IN BOUNDING REGION?

YES

NO

END

*FIG. 4*

13

Europäisches
Patentamt

European
Patent Office

Office européen
des brevets

# EUROPEAN SEARCH REPORT

Application Number

EP 17 21 0078

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | Christian Sigg ET AL: "GPU-Based Ray-Casting of Quadratic Surfaces", Eurographics Symposium on Point-Based Graphics, 1 January 2006 (2006-01-01), XP055455516, DOI: 10.2312/SPBG/SPBG06/059-065 Retrieved from the Internet: URL:http://reality.cs.ucl.ac.uk/projects/quadrics/pbg06.pdf [retrieved on 2018-03-01] | 1-6, 9-15, 18-20 | INV. G06T15/06 G06T17/10 G06T15/08 |
| A | * abstract * * Sections 1-6 * ----- | 7,8,16, 17 | |
| X | CARSTEN STOLL ET AL: "Incremental Raycasting of Piecewise Quadratic Surfaces on the GPU", IEEE SYMPOSIUM ON INTERACTIVE RAY TRACING, IEEE, SALT LAKKE CITY UT, USA, 1 September 2006 (2006-09-01), pages 141-150, XP031008782, ISBN: 978-1-4244-0693-7 | 1,4,9, 10,13, 18-20 | |
| A | * abstract * * Sections 1-7 * ----- | 2,3,5-8, 11,12, 14-17 | TECHNICAL FIELDS SEARCHED (IPC) G06T |
| A | US 2007/097121 A1 (LOOP CHARLES T [US] ET AL) 3 May 2007 (2007-05-03) * paragraph [0001] - paragraph [0114] * * figures 1-10 * ----- | 1-20 | |

The present search report has been drawn up for all claims

| Place of search | Date of completion of the search | Examiner |
|---|---|---|
| Munich | 9 March 2018 | Ernst, Jens |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**EP 3 340 184 A1**

## ANNEX TO THE EUROPEAN SEARCH REPORT
## ON EUROPEAN PATENT APPLICATION NO.

EP 17 21 0078

09-03-2018

| Patent document cited in search report | Publication date | Patent family member(s) | Publication date |
|---|---|---|---|
| US 2007097121 A1 | 03-05-2007 | NONE | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SIGG, CHRISTIAN et al.** GPU-Based Ray-Casting of Quadratic Surfaces. SPBG, 2006